# EUROPEAN PATENT APPLICATION

(11) **EP 1 759 661 A1**
(43) Date of publication of application: **07.03.2007**
(21) Application number: 06018458.7
(22) Date of filing: 04.09.2006
(51) Int. Cl.: A61F 2/06

(54) **Method and apparatus for delivery of a treatment element in a blood vessel**

(30) Priority: 02.09.2005 US 219437
(71) Applicant: Medtronic Vascular, Inc., Santa Rosa, California 95403 (US)
(72) Inventor: Chu, Jack, Santa Rosa, California 95409 (US); Raze, Brian, Ham Lake, Minnesota 55304 (US)
(74) Representative: Zimmermann & Partner

(57) **Abstract**

An excluding device (500) is inserted in a vessel. An excluded space (530) is formed between a portion of a wall of the vessel (520) and a portion of an exterior surface of the excluding device (500). A treatment element is passed through a channel (510) of the excluding device (500) into the excluded space (530). The channel (510) is sealed using a cuff (560) or patch after the passing of the treatment element(s) such as fill material or drugs and drug releasing elements.

## Description

### BACKGROUND OF THE INVENTION

### Field of The Invention

The present invention relates generally to treatment of a blood vessel or aneurysm, and more particularly to structures and delivery of an element to assist in treatment of a blood vessel or aneurysm.

### Description of The Related Art

Vascular aneurysms are the result of abnormal dilation of a blood vessel, usually resulting from disease and/or genetic predisposition, which can weaken the arterial wall and allow it to expand. While aneurysms can occur in any blood vessel, most occur in the aorta and peripheral arteries, with the majority of aortic aneurysms occurring in the abdominal aorta, usually beginning below the renal arteries and often extending distally into one or both of the iliac arteries.

Aortic aneurysms are often treated in open surgical procedures where the diseased vessel segment is bypassed and repaired with an artificial vascular graft. While considered an effective surgical technique, conventional vascular graft surgery however, is frequently not advisable for elderly patients or those patients weakened from cardiovascular and other diseases.

An alternative treatment to the open surgical procedure is placement of an endovascular prosthesis, such as an endoluminal stent graft, inside the vessel to isolate the aneurysm from blood flow and subsequent pressure. Generally, endoluminal stent grafts are delivered to a desired location within a vessel using a catheter-based delivery technique. To deliver the endoluminal stent graft within an acceptable size for a blood vessel, endoluminal stent grafts are typically compressed and housed in a removable sheathing. The endoluminal stent graft is then inserted into a vessel via the catheter-based delivery technique, positioned in the vessel, and the sheath removed allowing the endoluminal stent graft to expand and contact the vessel walls. Conventionally, the proximal end of the endoluminal stent graft is referenced with respect to the end closest to the heart (via the length of blood traveled from the heart). Some endoluminal stent grafts further include openings or bifurcations to accommodate lateral branches off the main vessel.

Endoluminal stent grafts typically include a graft material attached to a stent structure. The graft material is generally formed into a tubular shape with a hollow lumen. The graft material is typically a material that channels blood through the graft lumen without excessive leakage of blood into the surrounding vessel, and thus the graft material is typically tightly woven.

The stent structure is attached to the graft material so that when the stent structure is expanded, the stent-graft forms a tubular shape. The stent structure is typically formed of stainless steel, nitinol or other materials capable of being expanded with the graft material to strengthen the walls of the vessel and/or to provide support for the graft material through the vessel, e.g., through the aneurysm section of the vessel. Some portions of the stent structure are attached at the ends of the graft material at the lumen openings to provide additional support or anchoring of the endoluminal stent graft in the vessel.

Unfortunately, many endoluminal stent grafts in the prior art suffered a number of technical problems with subsequent morbidity and mortality. To assist in the healing in of a stent graft, coating drugs on the surface of the stent graft has been investigated.

For example, a drug was coated on the surface of a coronary stent graft to help prevent restenosis. The benefit of this approach was that approximation of the coronary stent graft to the vessel wall to be treated. However, for an aneurysm, other than the proximal and distal attachment points, the surface of the stent graft is not in close proximity to the vessel wall that is to be treated. Thus, the drug on the surface of the stent graft was washed away or diluted before the drug could reach the vessel wall. As a result, a significant amount of the drug was required to effectively treat the vessel wall. The additional drug coating complicated the device coating, delivery and sterilization.

### SUMMARY OF THE INVENTION

The above-mentioned problems associated with treating a portion of a wall of a blood vessel are ameliorated by a structure according to claims 1 and 4. Further aspects, features, details and advantages of the present invention are apparent from the dependent claims, the specification and the accompanying drawings. In particular, placing a treatment element in direct contact with the wall portion is enabled. This allows a drug or drugs in the treatment element to be applied directly to the wall and therefore overcome the problems of washing away and dilution.

In one example, a structure used for applying the treatment element includes an excluding device having a sidewall with an interior surface and an exterior surface. A channel extends through the excluding device from an opening in the interior surface to an opening in the outer surface. In one family of structures, the channel has a diameter of less than 2 millimeters. In another family of structures, the channel has a diameter of at least 2 millimeters.

In one example, the structure includes a gate valve mechanism attached to the excluding device. In a closed position, the gate valve mechanism covers one of the opening in the interior surface and the opening in the outer surface.

In another example of the structure, the structure includes a stent graft cuff. Upon deployment of the stent graft cuff in the excluding device, the stent graft cuff covers the opening in the interior surface. In one example, the stent graft cuff includes a plurality of fibers affixed to an outer circumferential surface of the stent graft cuff. In another example, stent graft cuff includes a coating affixed to an outer circumferential surface of the stent graft cuff.

In still another example of the structure, the structure includes an expansible plug mounted in the channel. The expansible plug includes a radio opaque material. In still yet another example of the structure, the structure includes a stent graft plug mounted in the channel. In a further example of the structure, the structure includes a patch affixed to a portion of-the interior surface and covering the opening in the interior surface.

Hence, in these examples, the structure includes at least one channel sealing device selected from at least one of a gate valve mechanism attached to the excluding device; a stent graft cuff wherein upon deployment of the stent graft cuff in the excluding device, the stent graft cuff covers the opening in the interior surface; an expansible plug mounted in the channel; a stent graft plug mounted in the channel; and a patch affixed to a portion of the interior surface and covering the opening in the interior surface. The structure can also include combinations of these sealing devices.

The structure also includes a treatment element in the excluded space about the portion of the outer surface of the excluding device.

An excluding device is inserted in a vessel. An excluded space is formed between a portion of a wall of the vessel and a portion of an exterior surface of the excluding device. A treatment element is passed through a channel of the excluding device into the excluded space. The channel is sealed after the passing of the treatment element.

### BRIEF DESCRIPTION OF THE DRAWINGS

FIG. 1 illustrates one example of a blood vessel with an excluding device and a treatment element positioned on a portion of a wall of the blood vessel.

FIG. 2A illustrates a stent graft with a channel extending through a sidewall of the stent graft to provide communication between a lumen formed by the stent graft and an excluded space.

FIG. 2B illustrates the stent graft of Fig. 2A with a first treatment element substantially filling the excluded space.

FIG. 2C illustrates the stent graft of Fig. 2A with a second treatment element substantially filling the excluded space.

FIG. 3A illustrates a stent graft with a channel extending through a sidewall of the stent graft to provide communication between a lumen formed by the stent graft and an excluded space and a gate valve mechanism mounted in the stent graft with the gate open.

FIG. 3B illustrates the stent graft of Fig. 3A with a treatment element substantially filling the excluded space.

FIG. 3C illustrates the stent graft of Fig. 3A with a treatment element substantially filling the excluded space and the gate closed.

FIG. 4A illustrates a first example of a stent graft cuff.

FIG. 4B illustrates a second example of a stent graft cuff.

FIG. 5 illustrates a stent graft with a channel extending through a sidewall of the stent graft to provide communication between a lumen formed by the stent graft and an excluded space; a treatment element substantially filling the excluded space; and a deployed stent graft cuff.

FIG. 6 illustrates a stent graft with a channel extending through a sidewall of the stent graft to provide communication between a lumen formed by the stent graft and an excluded space; a treatment element substantially filling the excluded space; and an expansible plug mounted in the channel.

Fig. 7A illustrates a stent graft plug.

FIG. 7B illustrates a stent graft with a channel extending through a sidewall of the stent graft to provide communication between a lumen formed by the stent graft and an excluded space; a treatment element substantially filling the excluded space; and the stent graft plug of Fig. 7A mounted in the channel.

FIG. 8 illustrates a stent graft with a channel extending through a sidewall of the stent graft to provide communication between a lumen formed by the stent graft and an excluded space; a treatment element substantially filling the excluded space; a patch covering an opening of in the channel; and a balloon catheter used to deploy the patch.

FIG. 9 illustrates a stent graft with a channel extending through a sidewall of the stent graft to provide communication between a lumen formed by the stent graft and an excluded space, and a plurality of radio-opaque markers mounted about the channel.

FIG. 10 illustrates one example of a method for delivery of a treatment element in a blood vessel.

Common reference numerals are used throughout the drawings and detailed description to indicate like elements.

### DETAILED DESCRIPTION

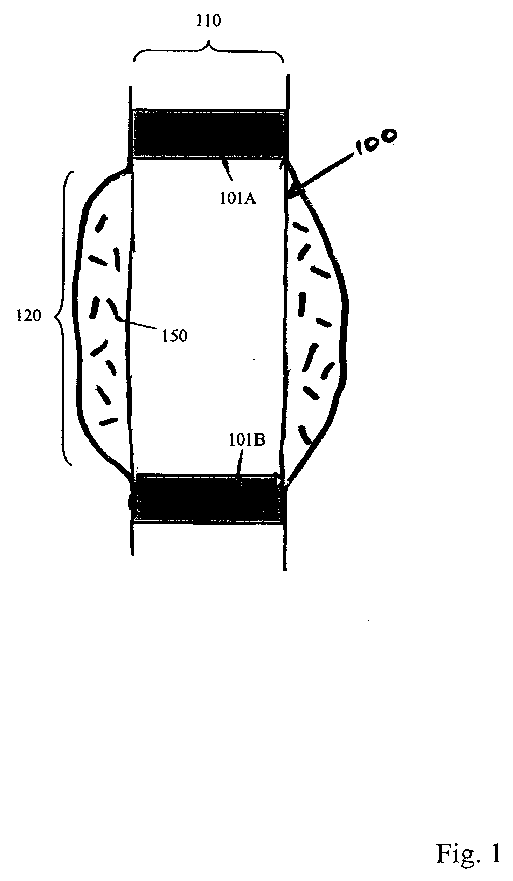

FIG. 1 illustrates one example of an exclusion device 100, including part 101A and part 101B, positioned in a blood vessel 110 to exclude blood from a region 120 of blood vessel 110 that is to be treated. A channel to region 120 is formed between part 101 A and part 101B. After exclusion device 100 is in place, a delivery system (not shown) is used to place treatment element(s) 150 adjacent to region 120, or to inject treatment element(s) 150 adjacent to region 120. Treatment element(s) 150 is shown spaced apart from region 120 for clarity only, but in practice, treatment element(s) 150 is placed on region 120, i.e., all or parts of treatment element(s) 150 are in contact with region 120. As is known to those of skill in the art, irregularities in the wall of region 120 may prevent a continuous direct contact between treatment element(s) 150 and region 120.

Treatment element(s) 150 includes an effective amount of a drug to treat region 120. Treatment element(s) 150 includes, for example, a drug, a drug impregnated material, a drug impregnated coating on a material, a drug encapsulated in a carrier, or a time-release encapsulated drug.

Treatment element(s) 150 is secured to region 120 by an attachment mechanism, in one example, such as an anchor, a plurality of anchors, a hook, a plurality of hooks, tissue glue, or hydrogel. The attachment of treatment element(s) 150 to region 120 assures that the drug or drugs are applied directly to region 120. The close contact eliminates the prior art problems of dilution and ineffective dose levels.

In one example, the delivery system is a balloon catheter, and treatment element(s) 150 is attached to an outer surface of the balloon. When the balloon is expanded, treatment element(s) 150 contacts region 120 and is secured to region 120 by the attachment mechanism. In another example, parts 101A and 101B are dual occlusion balloons and treatment element(s) 150 is delivered via a catheter, for example.

Unlike the prior art devices that used a coating on the outside of a stent, for example, treatment element(s) 150 is positioned directly adjacent to treatment area 120. Thus, prior art problems with dilution and washing away of the drug and damage of the coating during delivery are ameliorated.

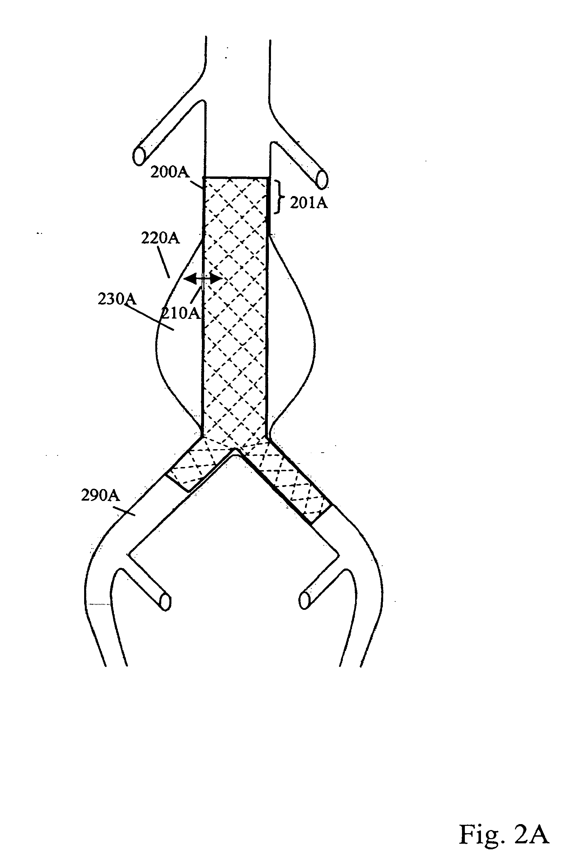

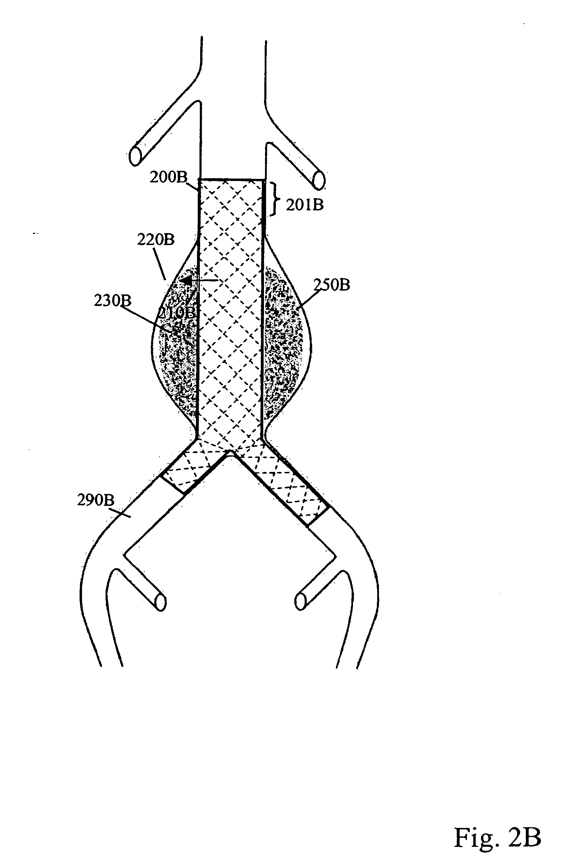

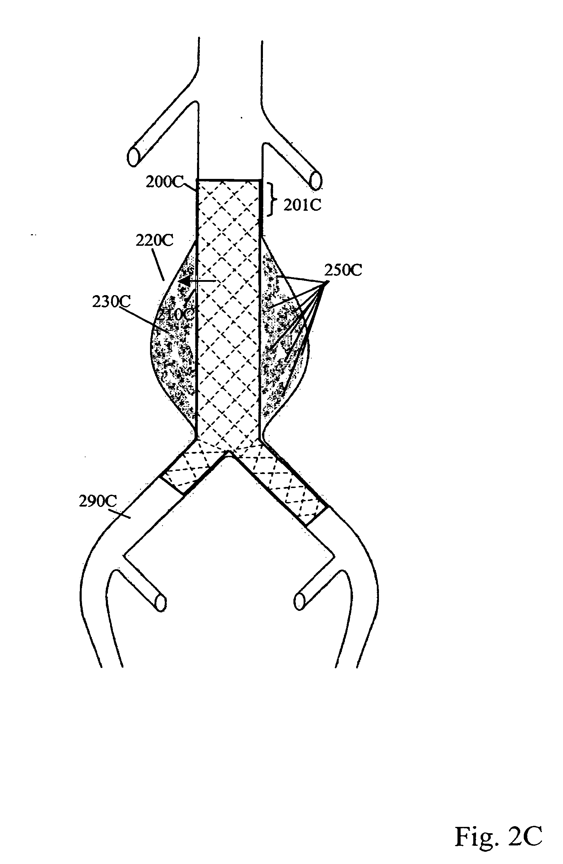

In general, exclusion device 100 also includes, but is not limited to, a stent, a stent graft, a film, and a balloon. Fig. 2 illustrates a cross-section of an exclusion device, i.e., a stent graft 200A, positioned in an aortic aneurysm 220A. Stent graft 200A is selected and positioned in aortic aneurysm 220A using known prior art techniques. In use, stent graft 200 excludes blood from excluded space 230A, which is bounded by a portion of an interior wall of the blood and a portion of an exterior wall of the excluding device as illustrated in Fig. 2A.

Stent graft 200A includes a channel 210A that extends through the wall of stent graft 200A so that excluded space 230A, which is located between the portion of the exterior surface of stent graft 200A and the portion of the interior wall of aneurysm 220A, communicates with the lumen formed by stent graft 200A. Excluded space 230A is an example of an excluded space. The excluding device, but for the channel, blocks blood flow into the excluded space. In this example, channel 210A has a diameter less than two millimeters, and is positioned below a neck 201A of aneurysm 220A. Channel 210A is one example of a mechanism for communicating between the excluded space and a lumen formed by the excluding device. The channel provides an opening to deliver filler material or drugs.

A catheter or wire is delivered through body lumen 290B, for example and passes through channel 210B. The catheter is used to deliver a treatment element 250B (Fig. 2B) into excluded space 230A. In Figs. 2A and 2B, elements with the same reference number, but a different letter in the reference numeral are equivalent elements if a description is not provided for the element with respect to Fig 2B. Treatment element 250B is any one of a liquid, foam, and a gel like material, for example. Treatment element 250B can be a drug, drugs, or a blend of a drug or drugs with a filler material.

Treatment element 250B substantially fills excluded space 230B and so prevents the space from filling with blood if a leak should develop around neck 201B of stent graft 200B for example. Here, substantially fills means that most but possibly not the entirety of excluded space 230B is filled. Depending on the form of treatment element 250B, there may be some small voids left that are not practicable to fill.

If treatment element 250B has adhesive characteristics, e.g., is a glue, treatment element 250B may help to prevent migration of stent graft 200B and provides some strengthening of the vessel wall by adhering the vessel wall to stent graft 200B. Finally, the drug or drugs in treatment element 250B are chosen, for example, so that when the drug leaches out of treatment element 250B, any blood in excluded space 230B is coagulated, medication is applied to the vessel wall, any other needed treatment function is achieved, or any combination of these functions.

Fig. 2C illustrates excluded space 230C substantially filled with a different treatment element 250C that has been delivered through artery 290C, through stent graft 200C, and channel 210C to excluded space 230C. In Figs. 2A and 2C, elements with the same reference number, but a different letter in the reference numeral are equivalent elements if a description is not provided for the element with respect to Fig 2C.

In this example, treatment element 250C is a plurality of pellets. Alternatively, or in combination, treatment element 250C could be a plurality of beads. The size and shape of the pellets and/or beads are selected to facilitate delivery to and passage through channel 210C into excluded space 230C. As indicated above, the type of drug included in the treatment element and the release pattern of the drug from the treatment element is selected to achieve the desired medication levels over the time frame of interest.

Channel 210A, 210B, 210C in stent graft 200A, 200B, 200C, as described above, has a diameter less that two millimeters. Thus, after passage of the treatment element though the channel, the channel is not sealed with a sealing device, because the channel is self-sealing, e.g., thrombosis form that seal the channel.

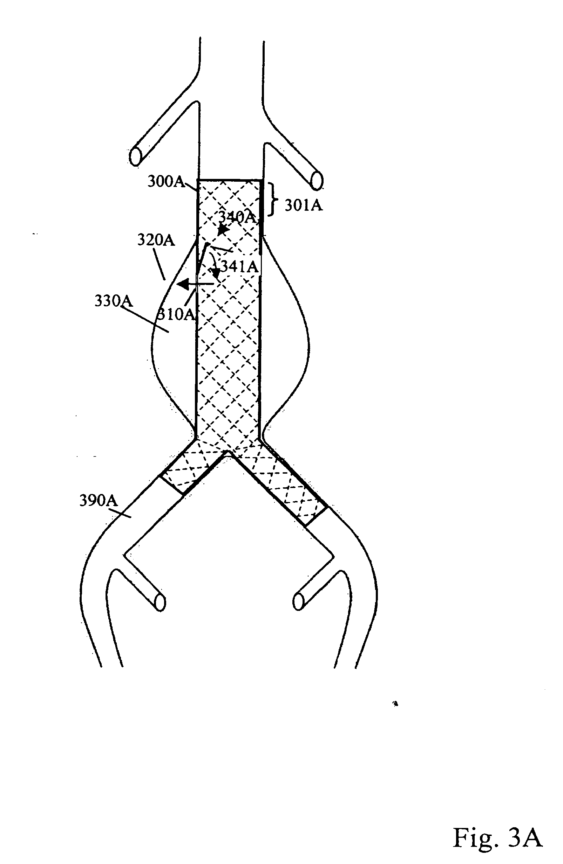

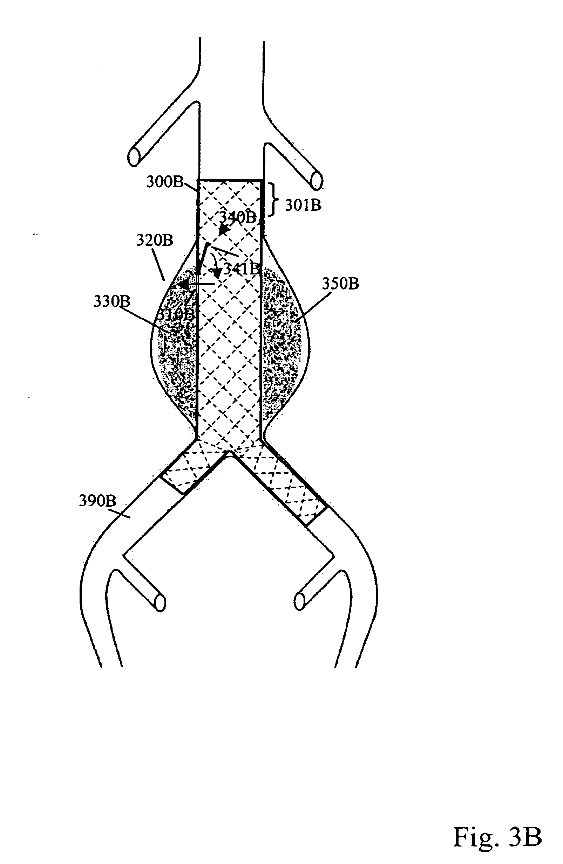

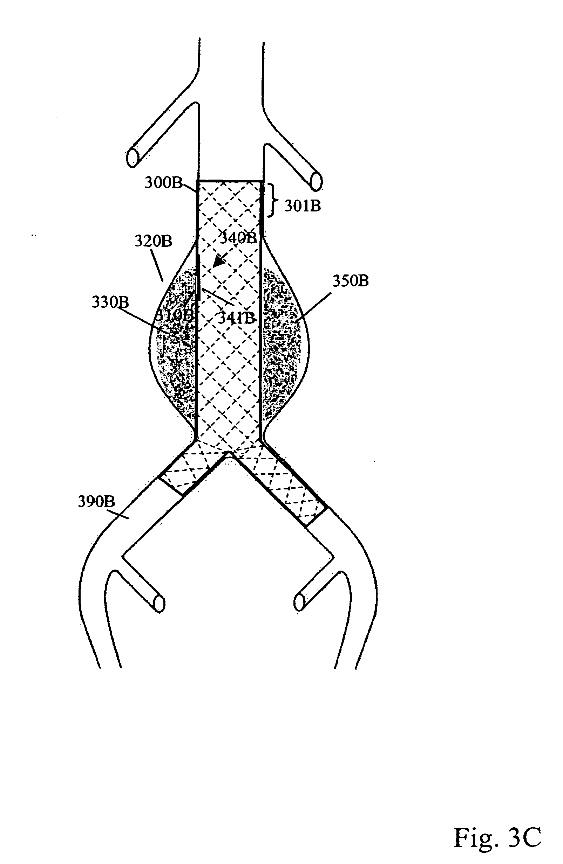

Fig. 3 illustrates a cross-section of an exclusion device 300A, i.e., a stent graft 300A, positioned in an aortic aneurysm 320A. Stent graft 300A is selected and positioned in aortic aneurysm 320A using known prior art techniques. In use, stent graft 300A excludes blood from excluded space 330A.

Stent graft 300A include a channel 310A that extends through the wall of stent graft 300A so that excluded space 330A, which is located between a portion of the exterior surface of stent graft 300A and a portion of the interior wall of aneurysm 320A, communicates with the lumen formed by stent graft 300A. In this example, channel 310A has a diameter greater than two millimeters, and is positioned below a neck 301A of aneurysm 320A. Channel 310A is one example of a mechanism for communicating between the excluded space and a lumen.

Stent graft 300A also includes a gate valve mechanism 340. In this example, a gate 341 of gate valve mechanism 340, sometimes referred to as a movable flab, is illustrated in an open position. Gate 341 can be temporarily held open in a variety of ways. For example, gate 341 can be temporarily glued to the interior surface of stent graft 300A, a band can be inserted to hold gate 341 open, or a balloon catheter could be used.

A catheter or wire is delivered through body lumen 390B, for example and passes through channel 310A. The catheter is used to deliver a treatment element 350B (Fig. 3B) into excluded space 330B. In Figs. 3A and 3B, elements with the same reference number, but a different letter in the reference numeral are equivalent elements if a description is not provided for the element with respect to Fig 3B. Treatment element 350B is any one of a liquid, foam, a gel like material, pellets and beads, for example. Treatment element 350B can be a drug, drugs, or a blend of a drug or drugs with a filler material.

Treatment element 350B substantially fills excluded space 330B and so prevents the excluded space from filling with blood if a leak should develop around neck 301 B of stent graft 300B for example. Also, if treatment element 350B has adhesive characteristics, e.g., is a glue, treatment element 350B may help to prevent migration of stent graft 300B and provides some strengthening of the vessel wall by adhering the vessel wall to stent graft 300B. Finally, the drug or drugs in treatment element 350B are chosen, for example, so that when the drug leaches out of treatment element 350B, any blood in excluded space 330B is coagulated, medication is applied to the vessel wall, any other needed treatment function is achieved, or any combination of these functions.

After treatment element 350B is inserted and the delivery catheter or wire is removed, gate 341 is closed and to seal the opening of channel 310B in the interior wall of stent graft 300B. In one example, gate 341 is fixed to the interior wall of stent graft 300B with a glue or some other means for attaching gate 341 to the interior wall. In this position, gate 341 seals the opening of channel 310B so that blood cannot flow through channel 310B.

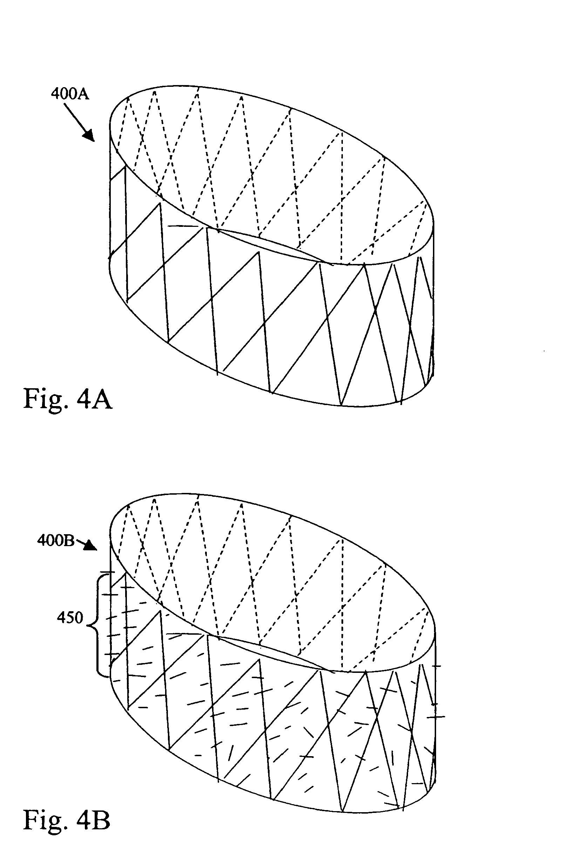

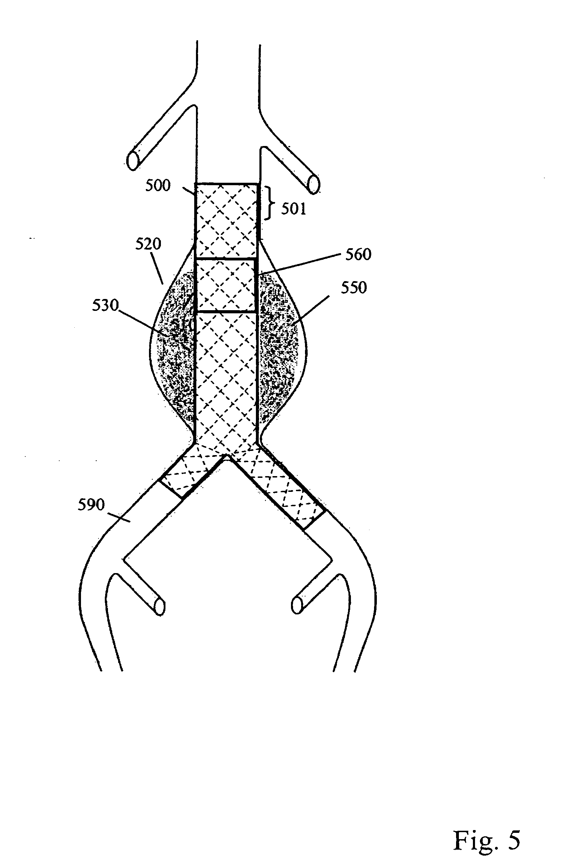

In another embodiment is stent graft cuff 400 (Fig. 4A) is used to seal the opening. After stent graft 500 (Fig. 5), with a channel 510, in inserted in aneurysm 520 and treatment element 550 is placed in excluded space 530, a stent graft cuff 560, for example, stent graft cuff 400A, is delivered, for example, using a catheter and deployed in stent graft 500, as illustrated in Fig. 5. In this example, channel 510 has a diameter greater than two millimeters.

Stent graft cuff 560 is made of materials equivalent to or the same as the materials used to make stent graft 500. Stent graft cuff 560 is sized to fit within and contact the inner wall of stent graft 500 on deployment. A length of stent graft cuff 560 is selected to assure that the opening of channel 510 is blocked, and to facilitate delivery and deployment. In one example, an outer circumferential surface of stent graft cuff 400A (Fig. 4A) is coated with an adhesive, or other sealant, to promote contact of the outer circumferential surface of stent graft cuff 400A with the interior circumferential surface of stent graft 500 (Fig. 5) to provide a more positive sealing of channel 510.

In another embodiment, stent graft cuff 400B (Fig. 4B) has fibers, or sutures 450 attached to an outer circumferential surface of stent graft cuff 400B. The length and size of the fibers as well as the density of the fibers is selected to promote the formation of thrombosis, which in turn aid in blocking blood flow through channel 510. Optionally, fibers 450 can be coated with or impregnated with a chemical that further promotes the formation of thrombosis. Stent graft cuff 400B is deployed and used as described for stent graft cuff 560.

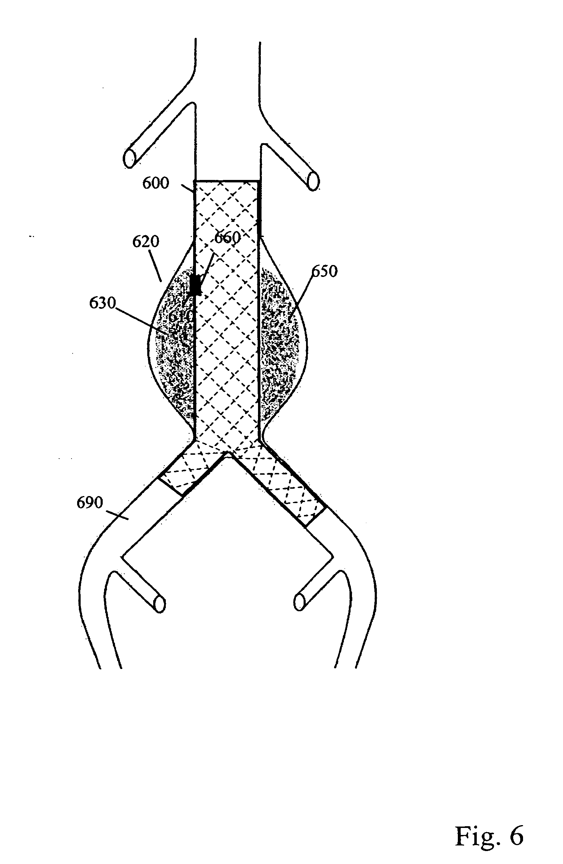

In another example, after stent graft 600 (Fig. 6), with a channel 610, is in inserted in aneurysm 620 and treatment element 650 is placed in excluded space 630, an expansible plug 660 is delivered using a catheter through artery 690 and inserted into channel 610. In this example, channel 610 has a diameter greater than two millimeters.

Expansible plug 660 is made from a polymer foam, e.g., PVA (polyvinyl alcohol), PU (polyurethane), PE (polyethylene), PP (polypropylene), or silicone. In one example, expansible plug 660 is radio opaque to assist in positioning plug 660 in channel 610. The radio-opacity of expansible plug 660 is enhanced by blending the polymer foam with a radio-opaque material or materials such as gold, silver, tantalum oxide, tantalum platinum, platinum/iridium alloy, or tungsten.

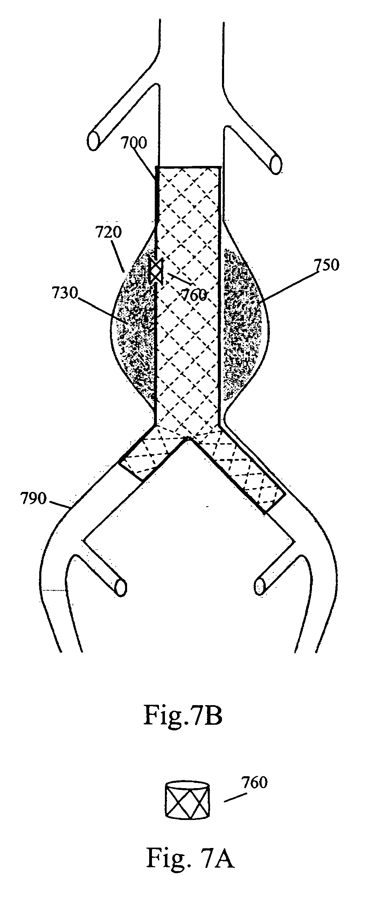

In yet another example, after stent graft 700 (Fig. 7), with a channel 710, is in inserted in aneurysm 720 and treatment element 750 is placed in excluded space 730, a stent graft plug 760 is delivered using a catheter through artery 790 and inserted into channel 710. In this example, channel 710 has a diameter of at least two millimeters.

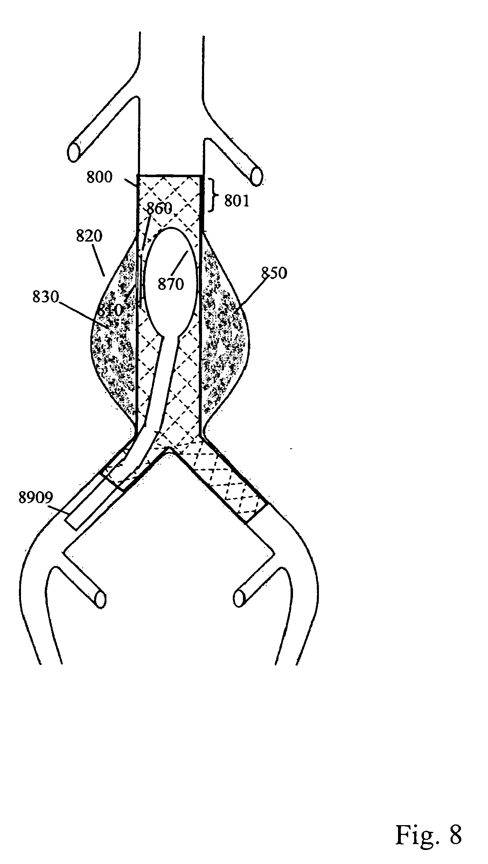

In still yet another example, after stent graft 800 (Fig. 8), with a channel 810, is in inserted in aneurysm 820 and treatment element 850 is placed in excluded space 830, a patch 860 is delivered using a balloon catheter 870, for example, through artery 890 and positioned on a portion of the inner circumferential surface of stent graft 800 to cover an opening of channel 810. In this example, channel 810 has a diameter of at least two millimeters. Patch 860 is affixed to the portion of the inner circumferential surface, e.g., glued to the surface. Alternatively, a glue or other filler material could be used to seal channel 810 instead of or in combination with patch 860.

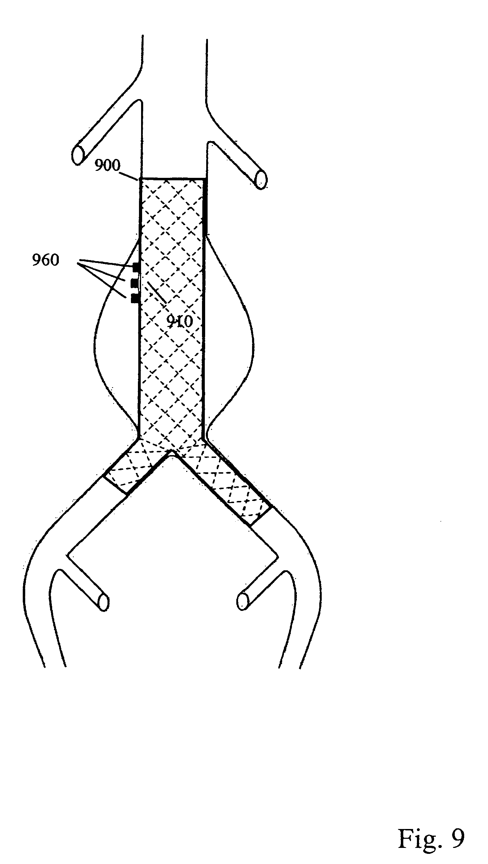

In the example of Fig. 9, stent graft 900 includes a plurality of radio-opaque markers 960 attached to stent graft 900 and oriented about channel 910. Plurality of radio-opaque markers 960 assist in passing the treatment element through channel 910 and in the subsequent sealing of channel 910.

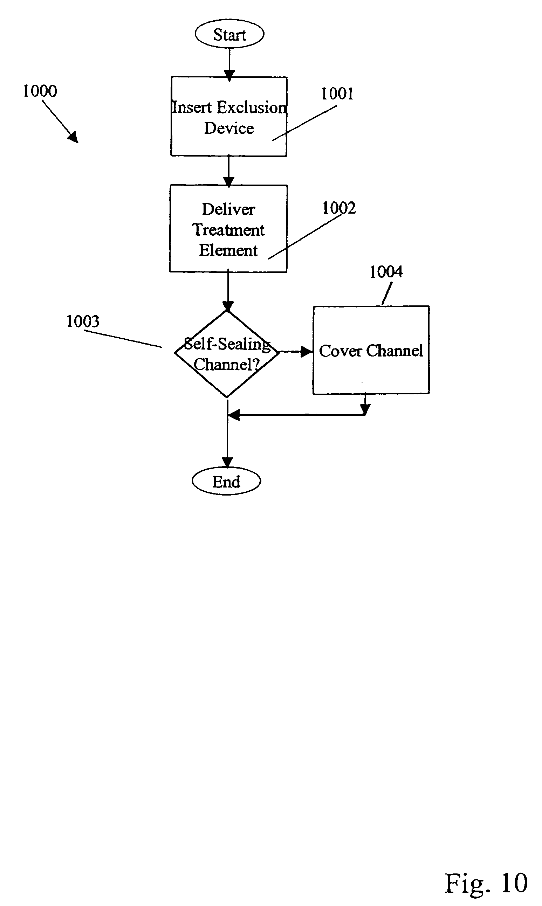

Fig. 10 is a process flow diagram for a method 1000 of treatment of a blood vessel wall, e.g., a wall of an aneurysm. In insert exclusion device operation 1001, an excluding device having an interior surface, an exterior surface and a channel extending from an opening in the interior surface to an opening in the exterior surface, i.e., through said excluding device, is inserted in the blood vessel using prior art techniques for inserting a stent or a stent graft. The channel does not have to be included in the excluding device at the time of insertion, but can be created in deliver treatment element operation 1002 as described below.

After insertion of the excluding device, deliver treatment element operation 1002 delivers a treatment element through the channel into an excluded space bounded by a portion of the blood vessel wall and a portion of the outer surface of the excluding device. If a needle is used to deliver the treatment element, the needle can puncture the wall of the excluding device to create the channel. In other examples, a wire or a catheter is used in operation 1002 to deliver the treatment element. As described above, the treatment element can take on a variety of forms and that description is incorporated herein by reference.

After delivery of the treatment element, a decision is made in self-sealing channel check operation 1003 whether a size of the channel is small enough that the channel will seal itself, e.g., via formation of thrombosis. If the channel will seal itself, no further action is required. However, if the channel is not self-sealing, cover channel operation 1004 is performed. In one embodiment, if the channel has a diameter of less than two millimeters, the channel is considered self-sealing.

In cover channel operation 1004, one of more of the sealing devices described above, or an equivalent of one or more of the device is used to block blood flow though the channel. For example, at least one sealing device selected from a group consisting of a gate valve mechanism attached to the excluding device; a stent graft cuff where upon deployment of the stent graft cuff in the excluding device, the stent graft cuff covers an opening in the interior surface of the excluding device; an expansible plug mounted in the channel; a stent graft plug mounted in the channel; and a patch affixed to a portion of the interior surface and covering the opening in the interior surface is used to seal the channel. Upon completion of operation 1004, the procedure is done.

Thus, the treatment element is delivered through the channel of the excluding device and placed directly in contact with the surface of the diseased vessel wall, and/or in the excluded space between the blood vessel wall and a portion of the outer circumferential surface of the excluding device. Thus, the channel functions as a communication port that is conveniently located near the place of treatment. This enhances the accuracy of the treatment.

According to an embodiment of the present invention, an excluding device is inserted into a vessel wherein an excluded space is formed between a wall of said vessel and a portion of an exterior surface of said excluding device. In a further step; a treatment element is passed through a channel extending from an interior surface of said excluding device to said exterior surface of said excluding device, into said excluded space sealing said channel after said passing, and a cuff is inserted within said excluding device. According to a further embodiment, radio-opaque markers may be used to locate said channel.

In the prior art, the torturosity of the interventional route to access the excluded space required a small and flexible device that could deploy at most a small treatment element, which typically was not an effective form of treatment. In contrast, the excluding device with a channel allows use of a large size treatment element relative to the prior art and allows use of a larger and more easily manipulated delivery system.

In the above example, for convenience of discussion, only a single channel was described. However, in view of this disclosure, an excluding device with a plurality of channels oriented about the device could be implemented by those of skill in the art. For example, channels could be implements so that centerlines of the channels were all in the same plane. Alternatively, channels could be located along the longitudinal direction of the excluding device. Similarly, a combination of the channel sealing devices could be used, e.g., a plug and a cuff. Therefore, the examples are illustrative only and are not intended to limit the invention to the specific examples described.

## Claims

1. A structure comprising:
an excluding device (200A) having a sidewall with an interior surface and an exterior surface and a channel extending through said excluding device from an opening in said interior surface to an opening in said outer surface, and
a stent graft cuff (400) wherein upon deployment of said stent graft cuff (400) in said excluding device, said stent graft cuff (400) covers said opening in said interior surface.

2. The structure of Claim 1 wherein said stent graft cuff (400B) further comprises:
a plurality of fibers (450) affixed to an outer circumferential surface of said stent graft cuff (400B).

3. The structure of Claim 1 or 2 wherein the length and/or the thickness and/or the density of said fibers (450) is selected to promote thrombosis.

4. The structure according to any of the preceding claims wherein said stent graft cuff (400A) further comprises:
a coating affixed to an outer circumferential surface of said stent graft cuff (400A).

5. The structure of claim 4, wherein the coating is an adhesive or a sealant to promote contact of the outer circumferential surface of stent graft cuff (400A) with the interior circumferential surface of the excluding device.

6. A structure comprising:
an excluding device (800) having a sidewall with an interior surface and an exterior surface and a channel (810) extending through said excluding device (800) from an opening in said interior surface to an opening in said outer surface, and
a patch (860) affixed to a portion of said interior surface and covering said opening in said interior surface.

7. The structure of any of the preceding claims further comprising at least one radio opaque marker (960) attached to said excluding device (900) about said channel (910).

8. The structure of any of the preceding claims further comprising:
a treatment element (150) in an excluded space about a portion of said outer surface.
